# FASCICULE DE BREVET EUROPEEN

(11) **EP 3 820 545 B1**
(45) Date de publication et mention de la délivrance du brevet: **05.11.2025**
(21) Numéro de dépôt: 19756219.2
(22) Date de dépôt: 11.07.2019
(51) Int. Cl.: A61M 5/145, A61M 39/28

(54) **DISPOSITIF D'INJECTION DE PRODUIT FLUIDE**
FLUIDPRODUKTINJEKTIONSVORRICHTUNG
FLUID PRODUCT INJECTION DEVICE

(30) Priorité: 13.07.2018 FR 1856480
(43) Date de publication de la demande: 19.05.2021
(73) Titulaire: Aptar France SAS, 27110 Le Neubourg (FR)
(72) Inventeur: FABIEN, David, 22130 CORSEUL (FR)
(74) Mandataire: CAPRI
(86) Numéro de dépôt international: PCT/FR2019/051744
(87) Numéro de publication internationale: WO 2020/012130

(56) Documents cités:
- EP-A1- 2 754 459
- WO-A1-2014/062160
- WO-A1-97/36623
- JP-A- 2004 290 455
- US-A- 4 909 710

## Description

La présente invention concerne un dispositif d'injection de produit fluide.

Les dispositifs d'injection de produit fluide sont bien connus. Ils comportent notamment les autoinjecteurs, dans lesquels le contenu d'un réservoir, généralement une seringue, est automatiquement injecté au moyen d'un système d'actionnement comprenant généralement un ressort chargé et qui lors du déclenchement va déplacer un piston dans le réservoir pour injecter le produit fluide.

Ces dispositifs de l'art antérieur peuvent présenter des problèmes, notamment lorsque les volumes à distribuer sont importants, lorsque le produit fluide est relativement visqueux ou lorsque plusieurs produits fluides doivent être associés dans un même traitement. Ainsi par exemple, les injecteurs de produits visqueux sont généralement peu compacts, lourds et volumineux, en particulier lorsqu'ils contiennent plusieurs réservoirs. De plus, le ou les produits fluides contenu(s) dans le ou les réservoir(s) sont généralement en contact avec de nombreux matériaux différents entre la sortie du réservoir et l'aiguille d'injection, ce qui peut présenter des risques de contamination potentielle du produit fluide. Par ailleurs, ces dispositifs complexes et intégrant souvent de l'électronique sont généralement jetables dans leur ensemble après utilisation.

Les documents JP2004290455, EP2754459, US4909710, WO9736623, WO2014062160, EP2179754 et US2007088271 décrivent des dispositifs de l'état de la technique.

La présente invention a pour but de fournir un dispositif d'injection qui ne reproduit pas les inconvénients susmentionnés.

La présente invention a aussi pour but de fournir un dispositif d'injection qui permet la distribution de produit fluide, même à des volumes et/ou viscosités importants.

La présente invention a également pour but de fournir un dispositif d'injection de produit fluide qui est compact et peu encombrant.

La présente invention a aussi pour but de fournir un dispositif d'injection de produit fluide qui comporte une partie réutilisable et/ou recyclable séparément.

La présente invention a également pour but de fournir un dispositif d'injection de produit fluide qui assure un contact du produit fluide à distribuer avec un nombre minimal de matériaux différents, tous adaptés au transport de produit fluide pharmaceutique.

La présente invention a également pour but de fournir un dispositif d'injection de produit fluide qui est simple et peu coûteux à fabriquer et à assembler.

La présente a donc pour objet un dispositif d'injection de produit fluide tel que décrit dans la revendication 1. Des modes de réalisation avantageux sont décrits dans les revendications dépendantes.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en perspective éclatée d'un dispositif d'injection automatique selon un mode de réalisation avantageux,
- la figure 2a est une vue schématique en perspective similaire à celle de la figure 1, en cours d'assemblage,
- la figure 2b est une vue schématique en perspective similaire à celle de la figure 2a, montrant une variante de réalisation,
- la figure 3 est une vue schématique en perspective similaire à celle de la figure 2, après assemblage et avant utilisation,
- la figure 4 est une vue schématique en perspective similaire à celle de la figure 3, en cours d'application sur le site d'injection,
- la figure 5 est une vue schématique partielle en perspective découpée du système de piquage de l'aiguille d'injection,
- la figure 6 est une vue schématique partielle en perspective éclatée d'un module sélecteur selon un mode de réalisation avantageux,
- la figure 7 est une vue schématique partielle en perspective du module sélecteur de la figure 6,
- la figure 8 est une vue schématique partielle en perspective d'un module de réservoir selon un mode de réalisation avantageux,
- les figures 9 et 10 sont des vues schématiques partielles en perspective montrant par transparence le fonctionnement du module sélecteur des figures 6 et 8,
- les figures 11 et 12 sont des vues schématiques partielles en perspective découpée montrant en coupe le fonctionnement du module sélecteur des figures 6 et 8,

Avantageusement, ledit module électronique est réutilisable et assemblé de manière amovible sur le dispositif.

Ces caractéristiques et avantages et d'autres apparaîtront plus clairement au cours de la description détaillée suivante, faite en référence aux dessins joints, donnés à titre d'exemples non limitatifs, et sur lesquels :

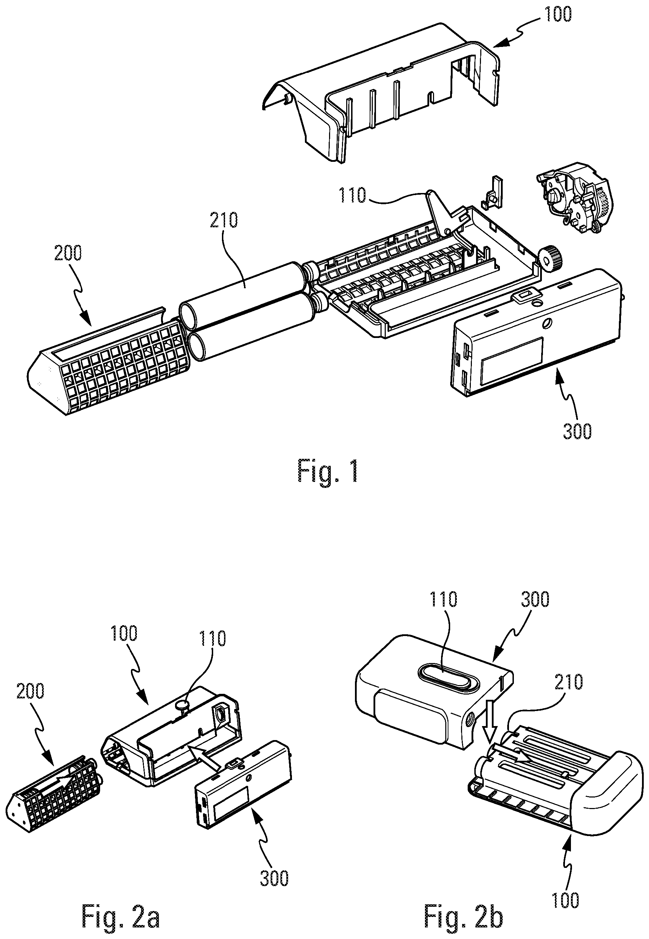

- la figure 1 est une vue schématique en perspective éclatée d'un dispositif d'injection automatique selon un mode de réalisation avantageux,
- la figure 2a est une vue schématique en perspective similaire à celle de la figure 1, en cours d'assemblage,
- la figure 2b est une vue schématique en perspective similaire à celle de la figure 2a, montrant une variante de réalisation,

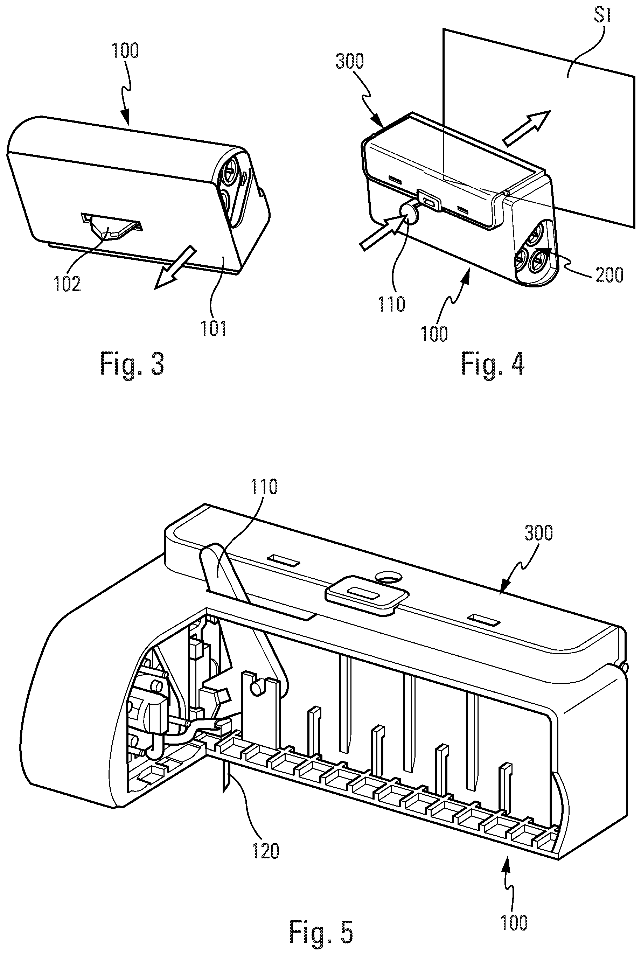

- la figure 3 est une vue schématique en perspective similaire à celle de la figure 2, après assemblage et avant utilisation,
- la figure 4 est une vue schématique en perspective similaire à celle de la figure 3, en cours d'application sur le site d'injection,
- la figure 5 est une vue schématique partielle en perspective découpée du système de piquage de l'aiguille d'injection,

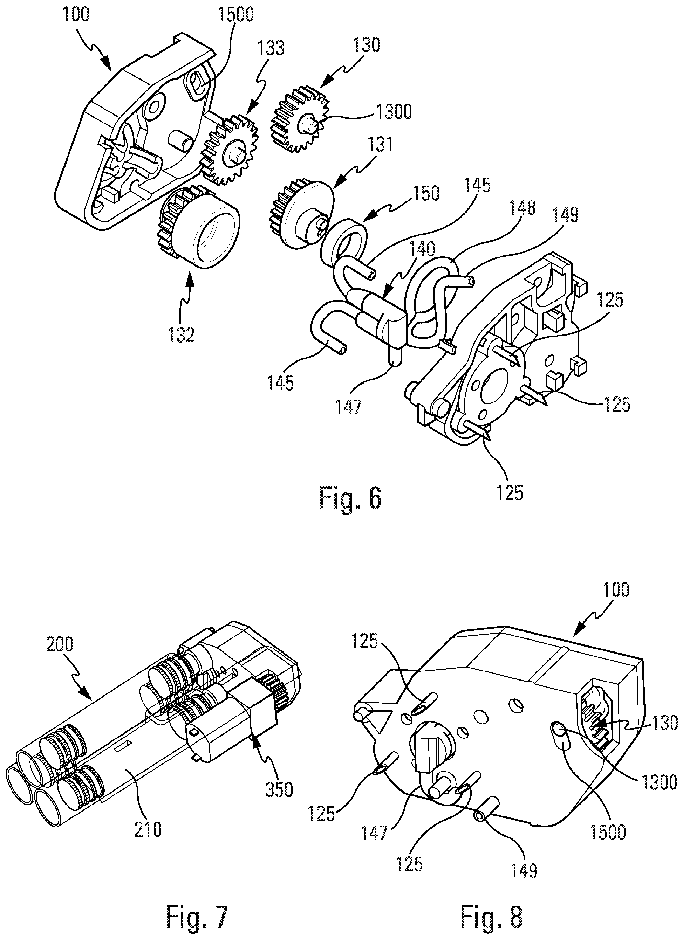

- la figure 6 est une vue schématique partielle en perspective éclatée d'un module sélecteur selon un mode de réalisation avantageux,
- la figure 7 est une vue schématique partielle en perspective du module sélecteur de la figure 6,
- la figure 8 est une vue schématique partielle en perspective d'un module de réservoir selon un mode de réalisation avantageux,

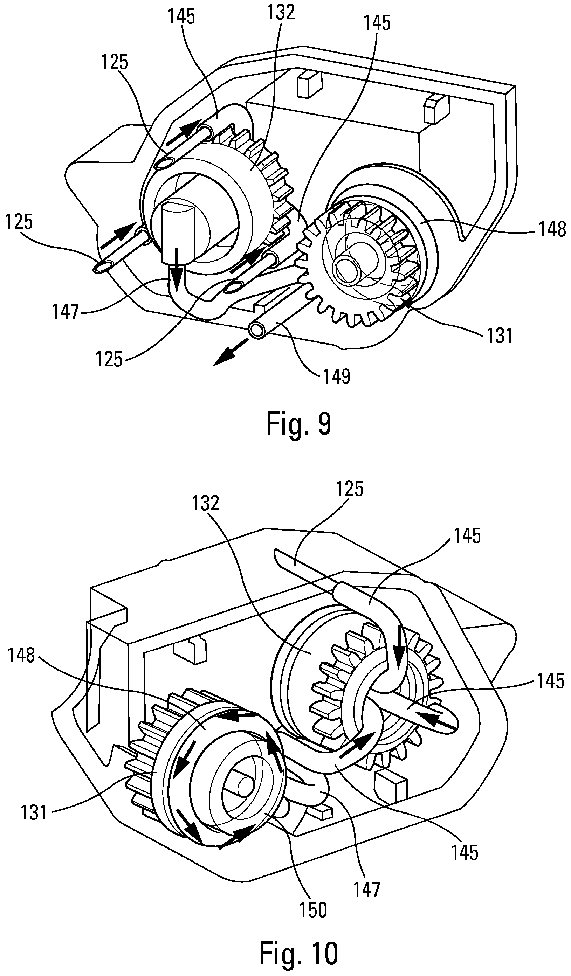

- les figures 9 et 10 sont des vues schématiques partielles en perspective montrant par transparence le fonctionnement du module sélecteur des figures 6 et 8,

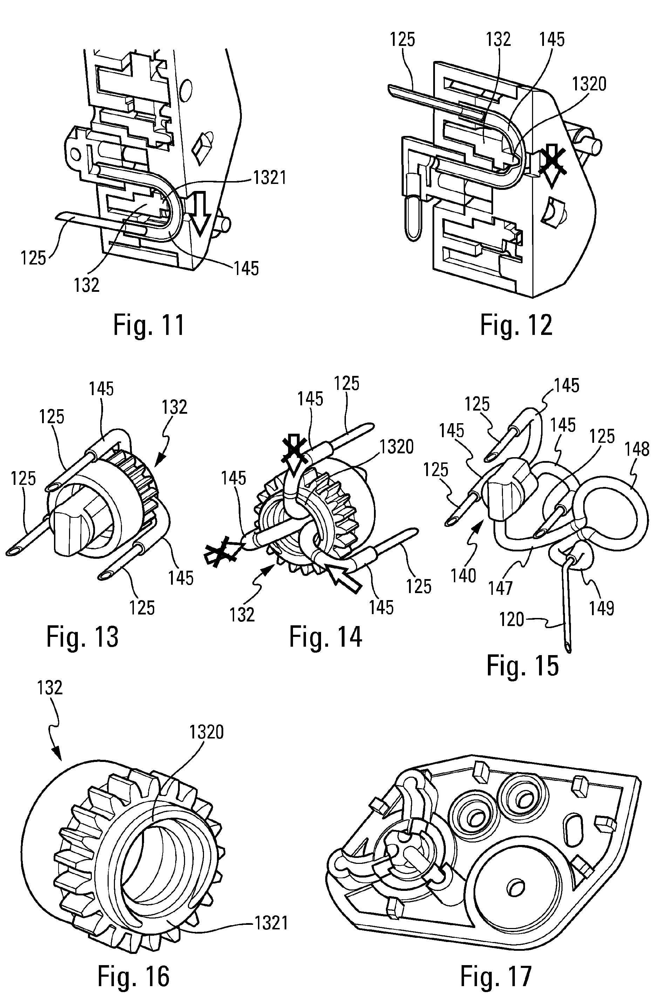

- les figures 11 et 12 sont des vues schématiques partielles en perspective découpée montrant en coupe le fonctionnement du module sélecteur des figures 6 et 8,
- les figures 13 et 14 sont des vues schématiques partielles en perspective montrant l'anneau sélecteur du module sélecteur des figures 6 et 8, vue de devant et de derrière respectivement,
- la figure 15 est une vue schématique partielle en perspective de la tubulure du module sélecteur des figures 6 et 8,
- la figure 16 est une vue schématique en perspective de l'anneau sélecteur du module sélecteur des figures 6 et 8,
- la figure 17 est une vue schématique partielle en perspective de la partie arrière du module sélecteur des figures 6 et 8,

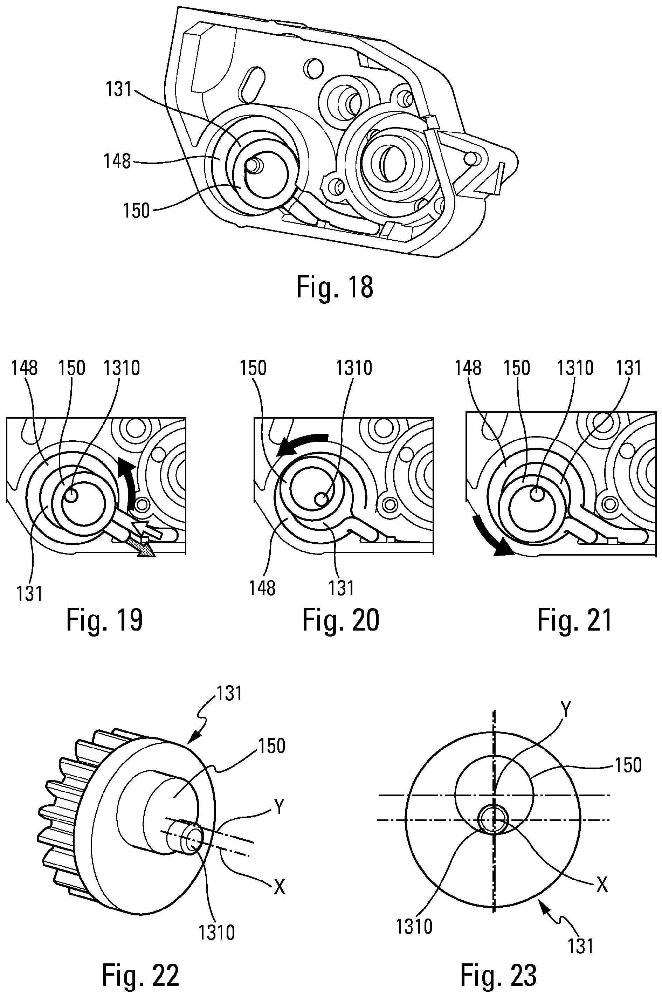

- la figure 18 est une vue schématique partielle en perspective découpée d'une pompe péristaltique selon un mode de réalisation avantageux,
- les figures 19 à 21 sont des vues schématiques montrant en coupe le fonctionnement de la pompe péristaltique de la figure 18,
- la figure 22 est une vue schématique en perspective du vilebrequin de la pompe péristaltique de la figure 18,
- la figure 23 est une vue schématique en coupe du vilebrequin de la pompe péristaltique de la figure 18,

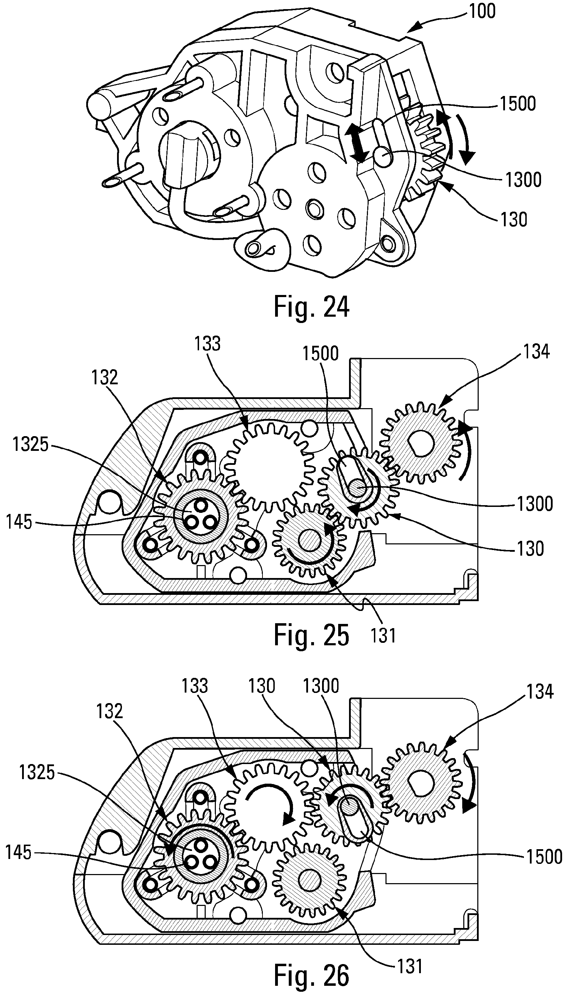

- la figure 24 est une vue schématique partielle en perspective découpée du module sélecteur selon un mode de réalisation avantageux,
- la figure 25 est une vue schématique en coupe de la roue flottante du module sélecteur de la figure 24, en mode actionnement de la pompe péristaltique,
- la figure 26 est une vue schématique en coupe de la roue flottante du module sélecteur de la figure 24, en mode sélecteur,

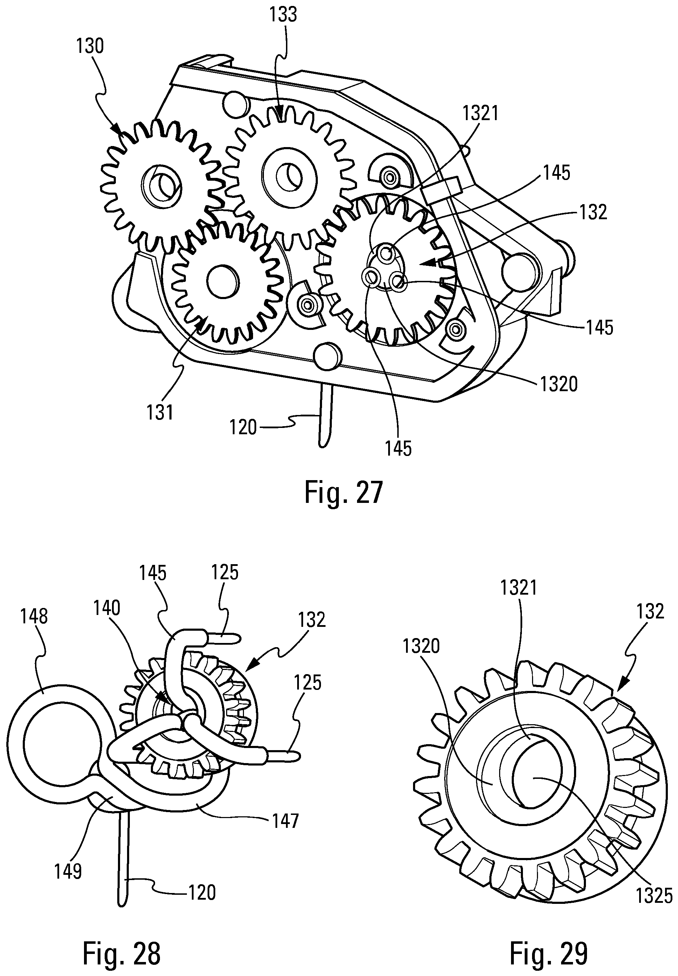

- la figure 27 est une vue schématique partielle en perspective découpée du module sélecteur selon un autre mode de réalisation avantageux,
- la figure 28 est une vue schématique partielle en perspective de la tubulure du module sélecteur de la figure 27,
- la figure 29 est une vue schématique partielle en perspective de l'anneau sélecteur du module sélecteur de la figure 27,

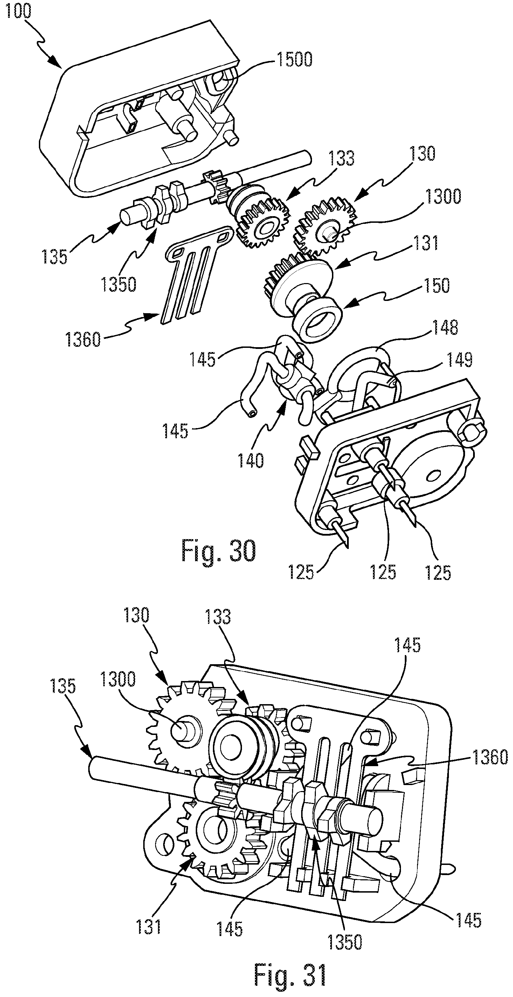

- la figure 30 est une vue schématique partielle en perspective éclatée d'un module sélecteur selon encore un autre mode de réalisation avantageux,
- la figure 31 est une vue schématique partielle en perspective découpée du module sélecteur de la figure 30,

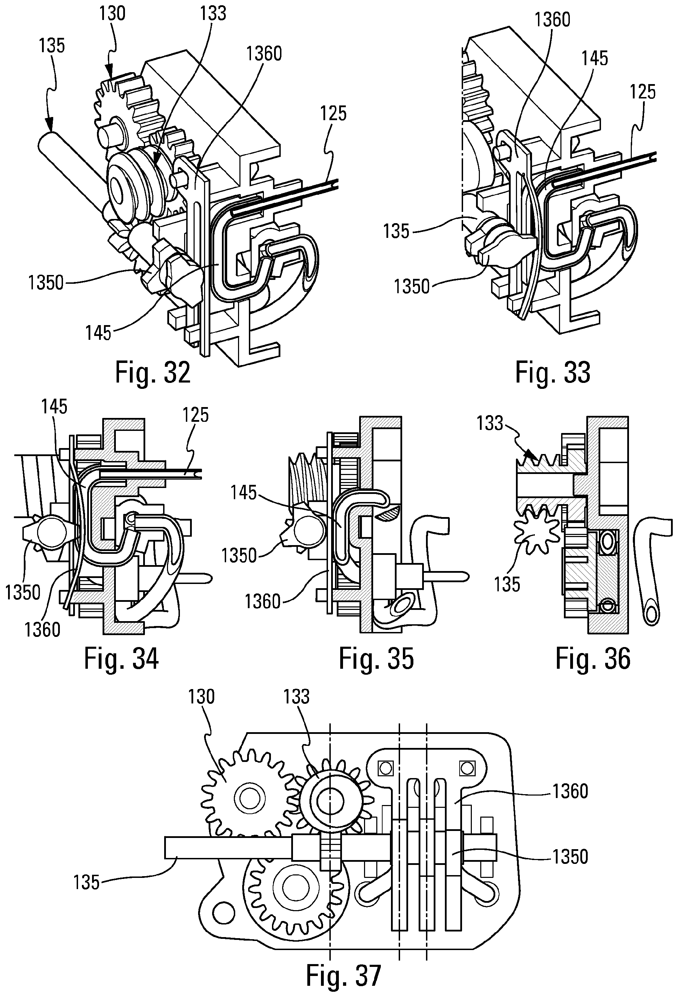

- les figures 32 et 33 sont des vues schématiques partielles en perspective découpée montrant le fonctionnement du module sélecteur des figures 30 et 31,
- les figures 34 à 36 sont des vues schématiques partielles en coupe montrant le fonctionnement du module sélecteur des figures 30 et 31,
- la figure 37 est une vue schématique en coupe similaire à la figure 31,

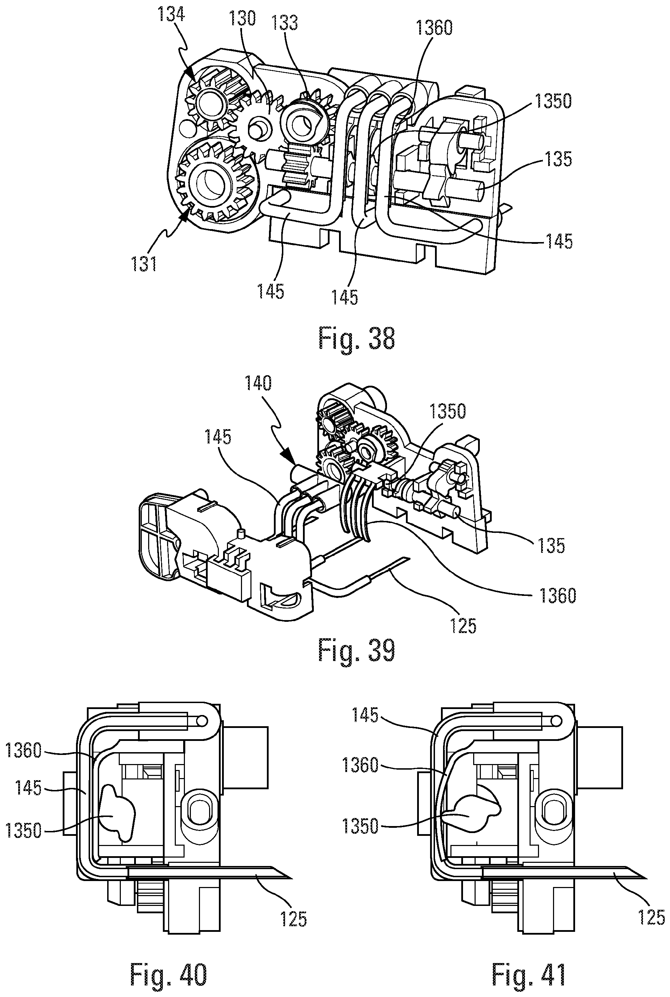

- la figure 38 est une vue schématique partielle en perspective découpée d'un module sélecteur selon encore un autre mode de réalisation avantageux,
- la figure 39 est une vue schématique partielle en perspective éclatée du module sélecteur de la figure 38, et
- les figures 40 et 41 sont des vues schématiques partielles en perspective découpée montrant le fonctionnement du module sélecteur des figures 38 et 39.

L'invention concerne un dispositif d'injection particulièrement adapté à distribuer des volumes relativement importants de produit fluide, typiquement de l'ordre de quelques millilitres, typiquement de 1 à 10 ml, par exemple 3 ml. Le dispositif de l'invention est aussi adapté à distribuer des produits fluides relativement visqueux.

Avantageusement, le dispositif comporte plusieurs modules. Ainsi, dans l'exemple des figures 1, 2a, 3 et 4, le dispositif comporte un module principal, ci-après appelé module sélecteur 100, un module réservoir 200 et un module électronique 300. Les modules sélecteur 100 et réservoir 200 sont de préférence jetables, alors que le module électronique 300 est de préférence réutilisable. Dans la variante de la figure 2b, les modules sélecteur 100 et réservoir 200 forment un seul module.

Le module réservoir 200 comporte ici trois réservoirs 210, avantageusement disposés en triangle, notamment pour des raisons de gain de place, mais il est entendu qu'un nombre quelconque de réservoirs peut être prévu, par exemple un seul réservoir, deux réservoirs ou plus de trois réservoirs. Lorsque plusieurs réservoirs 210 sont prévus, comme dans les exemples des figures, ceux-ci peuvent contenir des médicaments identiques ou différents. Dans les exemples des figures 2b et 38 à 41, les réservoirs 210, également au nombre de trois, sont disposés côte à côte, et non en triangle. De manière classique, chaque réservoir 210 peut comporter un piston, qui, lors de l'actionnement, sera déplacé dans ledit réservoir.

L'utilisation d'un dispositif à un ou plusieurs réservoirs permet de fournir notamment les avantages suivants:
- dispositif unique pour plusieurs types de produits fluides, qui peut nécessiter la distribution de volumes différents;
- possibilité de distribuer des cocktails ou mélange de plusieurs produits fluides;
- possibilités d'associer des agents réducteurs de douleur (anesthésiques, neutralisateur d'acidité, etc.) au médicament à injecter;
- possibilité d'avoir des fréquences de traitement de médicaments différents; par exemple une première séquence S1 avec la prise de plusieurs médicaments différents, suivie d'une seconde séquence S2 avec la prise d'un seul médicament, etc.;
- possibilité de standardiser le dispositif d'injection pour plusieurs types de traitement;
- diminution du coût de développement des dispositifs;
- possibilité d'ajustements dans la formulation du produit fluide.
- diverses formulations de produits fluides peuvent être logées dans un seul dispositif;
- réduction du nombre d'injections.

Les figures 2a, 3 et 4 illustrent les étapes successives lors de l'utilisation du dispositif.

Ainsi, le module électronique 300 et le module réservoir 200 sont d'abord assemblés sur le module sélecteur 100, comme visible sur la figure 2a. On peut prévoir d'abord l'assemblage du module électronique 300 ou au contraire d'abord l'assemblage du module réservoir 200. Avantageusement, on peut prévoir d'activer le module électronique 300 au moment de son assemblage, pour le faire passer d'un mode « veille » ou éteint, où il ne consomme pas ou peu d'énergie, à un mode « actif » dans lequel il est prêt à fonctionner. L'activation du module électronique 300 peut aussi se faire lors de l'assemblage du module réservoir 200, dans le cas où celui-ci est réalisé en dernier. Eventuellement, comme illustré sur la figure 3, le dispositif peut comporter un capteur 102 dans la surface qui sera appliquée sur le site d'injection SI, pour activer le module électronique seulement au moment où le dispositif est appliqué sur ledit site d'injection SI.

Dans la variante de la figure 2b, le dispositif pourrait ne comporter que deux modules, un module principal, regroupant le module sélecteur 100 et le module réservoir 200, et un module électronique 300.

Lorsque le dispositif est assemblé, le film de protection 101 prévu sur la face arrière du module sélecteur 100 est retiré (figure 3), et le dispositif est appliqué sur le site d'injection SI (figure 4), où il est maintenu par un adhésif adéquat, de manière connue.

L'utilisateur appuie alors sur un bouton d'actionnement 110 du module sélecteur 100 ou du module électronique 300 pour actionner le dispositif et injecter du produit fluide dans le site d'injection SI.

La commande du dispositif est avantageusement réalisée par le module électronique 300. Celui-ci comporte notamment une alimentation, notamment une pile ou un accumulateur, un micro-processeur, des moyens de stockage, des moyens de réception et/ou d'émission de signaux.

De préférence, le dispositif est autonome, mais il pourrait être commandé à distance, par transmission au module électronique des instructions de commande lors de l'actionnement du dispositif, en particulier la sélection et/ou la séquence du ou des réservoirs à distribuer, la vitesse de distribution, etc.

Le module électronique commande avantageusement un moteur 350 qui va actionner les éléments mobiles du dispositif pour réaliser un cycle d'actionnement.

Ces moyens électroniques du module électronique 300 ne sont pas décrits plus en détail ici, car s'ils participent au fonctionnement du dispositif, ils n'en forment pas des caractéristiques essentielles, et ils pourraient être réalisés d'une manière quelconque, bien connue du spécialiste de l'art.

En variante, on pourrait prévoir un système d'actionnement mécanique, par exemple utilisant un ou plusieurs ressorts, pour réaliser l'actionnement du dispositif en lieu et place du module électronique.

En fin d'injection, le dispositif est retiré du site d'injection SI, le module électronique 300 est retiré du dispositif, notamment pour pouvoir être réutilisé, et les modules sélecteur 100 et réservoir 200 sont jetés au rebut.

La figure 5 illustre un exemple de bouton d'actionnement 110, ici réalisé sous la forme d'un levier pivotant sur le corps du module sélecteur 100. Le pivotement dudit levier provoque d'une part l'insertion d'une aiguille d'injection 120 dans le site d'injection et l'actionnement du dispositif pour distribuer du produit fluide à travers ladite aiguille d'injection 120.

Avantageusement, le ou les réservoirs 210 sont obturés avant actionnement par une membrane formant septum, destinée à être percée par une aiguille d'amorçage 125 lors de l'actionnement. Dans l'exemple représenté sur les figures 7 et 8, le module sélecteur 100 comporte trois aiguilles d'amorçage 125, une pour chaque réservoir 210, pour réaliser l'amorçage en perçant la ou les membrane(s) lorsque le module réservoir 200 est assemblé dans le module sélecteur 100.

Si plusieurs réservoirs 210 sont utilisées, comme représenté dans l'exemple des figures 7 et 8, les aiguilles d'amorçage 125 de tous les réservoirs 210 sont couplées à une seule aiguille d'injection 120.

Le module sélecteur 100 va être décrit en référence à plusieurs modes de réalisation avantageux.

Avantageusement, le fonctionnement du module sélecteur 100 est le suivant :
- sélection du ou des réservoir(s) 210 à distribuer ;
- distribution du ou des médicament(s) contenu(s) dans le ou les réservoir(s) 210 sélectionné(s).

La sélection du ou des réservoir(s) 210 se fait par pincement/obstruction des tubes reliés à chaque réservoir.

La distribution du contenu du ou des réservoir(s) 210 sélectionnés se fait au moyen d'un système de distribution, de préférence réalisé sous la forme d'une pompe péristaltique 150, qui sera décrite plus amplement ci-après en référence aux figures 18 à 23.

Le module sélecteur 100 comporte avantageusement un actionneur rotatif unique 130 pourvu d'un système d'embrayage 131, 132, 133, 134, 135. Lorsque l'actionneur 130 tourne dans un premier sens de rotation, il active la sélection du ou des réservoir(s) 210 à distribuer, et lorsqu'il tourne dans le sens inverse, il actionne le système de distribution, à savoir la pompe péristaltique 150 dans l'exemple des figures 18 à 23. Avantageusement, c'est le moteur 350 du module électronique 300, visible sur la figure 7, qui fait tourner ledit actionneur 130.

Cet actionneur 130 comporte un axe central 1300 qui s'étend dans une ouverture oblongue 1500 du corps du module sélecteur 100.

Suivant le sens de rotation de l'actionneur 130, cet axe central 1300 va se translater d'un côté ou de l'autre de ladite ouverture oblongue 1500.

Du premier côté, comme visible sur la figure 26, l'actionneur 130 va coopérer avec un engrenage d'une roue de sélection 132, notamment via une roue intermédiaire 133, pour actionner la sélection du ou des réservoirs à distribuer.

De l'autre côté, comme visible sur la figure 25, l'actionneur 130 va coopérer avec un engrenage d'un vilebrequin 131, pour actionner la pompe péristaltique.

Dans les exemples des figures 25, 26 et 38, 39, une roue d'entrainement 134 est prévue pour faire tourner l'actionneur 130 dans l'un ou l'autre sens de rotation. Cette roue d'entrainement 134 peut être reliée au moteur. En variante, cette roue d'entrainement pourrait être omise, et le moteur relié directement à l'actionneur 130.

L'actionneur 130 est donc une roue flottante. Sa translation dans l'ouverture oblongue 1500 est réalisée en fonction de son sens de rotation et du fait des couples résistants des autres éléments rotatifs décrits ci-dessus.

Le module sélecteur 100 comporte également avantageusement un collecteur 140, qui comprend un tube 145 sortant de chaque réservoir 210. Chaque tube 145 est relié d'un côté à son réservoir 210 et de l'autre côté au système de distribution, en l'occurrence ici la pompe péristaltique 150 puis l'aiguille d'injection 120, via un ensemble de tubes 147, 148, 149.

Lorsqu'il y a plusieurs réservoirs 210, notamment trois comme dans les exemples des figures, les tubes 145 se rejoignent en aval du système de distribution, pour ensuite déboucher dans l'aiguille d'injection 120. Cet ensemble de tubes 145, 147, 148, 149 forme une tubulure, dont diverses variantes sont visibles notamment sur les figures 6, 15 et 30.

Avantageusement, le ou les tube(s) 145, 147, 148, 149 sont réalisés en un matériau compatible avec le ou les produit(s) fluide(s) à distribuer, par exemple les matériaux communément utilisés pour fabriquer des cathéters.

La sélection du ou des réservoirs 210 à distribuer lors de l'actionnement se fait avantageusement par pincement ou écrasement d'un ou plusieurs tubes 145 reliés aux réservoirs 210. Lorsqu'un tube 145 est « pincé », la circulation du fluide est empêchée dans ce tube, et le contenu du réservoir 210 respectif ne pourra pas circuler vers l'aiguille d'injection 120.

Le pincement du ou des tubes 145 est réalisé de préférence par des moyens de came formés sur une pièce mobile, notamment un organe rotatif.

La figure 11 montre un tube 145 non pincé, avec par conséquent une circulation de fluide dans ledit tube 145 qui est possible, alors que la figure 12 montre un tube 145 pincé ou écrasé, avec donc aucune circulation de fluide possible.

Dans l'exemple représenté sur les figures 11 à 16, une roue de sélection 132, qui fait partie du système d'embrayage de l'actionneur rotatif unique 130, comporte une came 1320 formée par une projection en arc de cercle, ladite came étant interrompue par un évidement 1321, comme visible sur la figure 16. Lorsque la came 1320 vient au contact d'un tube 145, elle le pince pour couper la circulation de fluide, alors que lorsque le tube 145 se trouve face audit évidement 1321, la circulation est possible.

Les figures 13 et 14 montrent un exemple dans lequel deux des trois tubes 145 sont pincés, et un seul tube 145 est « ouvert ». Lorsqu'on actionne la pompe, le médicament sera aspiré depuis le réservoir 210 relié audit tube 145 ouvert.

Bien entendu, d'autres mises en œuvre sont possibles, comme illustré sur les figures 27 à 41, qui montrent d'autres variantes de réalisation.

Ainsi, sur les figures 27 à 29, la came 1320 est formée de manière radiale dans un trou central 1325 de la roue de sélection 132. Cette came 1320 comporte une partie de plus grand diamètre 1321. Les tubes 145, au nombre de trois dans cet exemple aussi, passent à travers ce trou central 1325. Lorsqu'un tube 145 coopère avec la came 1320, il est pincé, alors que s'il coopère avec la partie de plus grand diamètre 1321, il n'est pas pincé.

Les figures 30 à 37 montrent un autre mode de réalisation. Ici, la roue de sélection est remplacée par un arbre de sélection rotatif 135, supportant plusieurs éléments de came 1350, trois dans l'exemple représenté. Un organe de pincement 1360 est prévu entre lesdits éléments de came 1350 et les tubes 145. Cet organe de pincement 1360 comporte avantageusement des lames flexibles, une pour chaque tube 145, qui sont déformées élastiquement par les éléments de came 1350 de l'arbre de sélection 135. Ceci évite le frottement des éléments de came sur les tubes, ce qui peut avoir comme inconvénient de les déformer et de rendre le pincement moins efficace.

Les figures 32 et 35 montrent un tube 145 non pincé, avec par conséquent une circulation de fluide dans ledit tube 145 qui est possible, alors que les figures 33 et 34 montrent un tube 145 pincé ou écrasé par une lame de l'organe de pincement 1360, elle-même pousse par un élément de came 1350 de l'arbre de sélection 135, avec donc aucune circulation de fluide possible.

L'exemple des figures 38 à 41 est très similaire à celui des figures 30 à 37, avec les trois réservoirs qui sont disposés côte à côte, comme illustré sur la figure 39 par les trois aiguilles d'amorçage 125 disposées de manière parallèle dans un même plan.

Dans les exemples représentés, l'organe de pincement 1360 forme une pièce monobloc, mais en variante on pourrait prévoir plusieurs organes de pincement séparés, chacun formé par une lame flexible.

Le système de distribution comporte de préférence une pompe péristaltique. Celle-ci comporte un anneau 150 qui tourne sur un vilebrequin 131 autour d'un axe de rotation Y désaxé par rapport à l'axe de rotation X dudit vilebrequin 131. De préférence, comme visible sur la figure 22, l'axe de rotation X du vilebrequin 131 est formé par un cylindre axial central 1310, autour duquel peut tourner l'anneau 150. Une partie de tube 148 qui va du collecteur 140 vers l'aiguille d'injection 120 s'étend autour dudit vilebrequin 131, de telle sorte que l'anneau 150, au fur et à mesure qu'il tourne autour de son axe de rotation désaxé Y, va tourner autour dudit cylindre axial central 1310 du vilebrequin 131 en déplaçant la compression le long du tube 148, générant ainsi la distribution du produit fluide qui passe dans ledit tube 148. Les figures19 à 21 illustrent le fonctionnement de la pompe péristaltique.

Lorsque la distribution du produit fluide est terminée, l'aiguille d'injection 120 est rétractée dans le dispositif, de préférence automatiquement. La fin de la distribution du produit fluide peut être identifiée par un contrôle mécanique et/ou logiciel.

Le mode de réalisation illustré sur les figures montre un dispositif adapté à comporter un, deux ou trois réservoirs 210. On peut prévoir l'utilisation de masques sur le module réservoir 200 qui, lorsque le dispositif est assemblé, sont perforés/pénétrés par la présence du réservoir. Lorsque le réservoir n'est pas présent, le masque agit pour sceller la branche respective de la tubulure, empêchant la fuite de médicament pendant la distribution.

Le mode de réalisation décrit fournit notamment les avantages suivants:
- la vitesse de distribution du produit fluide peut être ajustée pour optimiser les traitements individuels et peut également varier dans le temps;
- les réservoirs multiples permettent l'utilisation d'une combinaison de médicaments qui peuvent être distribués à des vitesses différentes et à des moments différents.

L'utilisation d'injections simultanées ou séquentielles peut être appliquée dans un système à plusieurs cartouches pour:
- réduire le débit de produit fluide et soulager la douleur du patient;
- permettre une amélioration de l'efficacité de certaines préparations de cocktail de médicaments.

La présente invention a été décrite en référence à plusieurs modes et variantes de réalisation avantageux, mais il est entendu qu'un homme du métier peut y apporter toutes modifications, sans sortir du cadre de la présente invention tel que défini par les revendications annexées.

## Revendications

1. Dispositif d'injection de produit fluide comportant un corps, destiné à venir au contact d'un site d'injection (SI), au moins un réservoir (210) de produit fluide, une aiguille d'injection (120) destinée à pénétrer dans ledit site d'injection (SI) pour y injecter le contenu d'un ou plusieurs réservoir(s) (210), une aiguille d'amorçage (125) associée à chaque réservoir (210) et destinée à pénétrer dans ledit réservoir (210) avant distribution du produit fluide, chaque réservoir (210) comportant un tube (145) relié d'un côté à son aiguille d'amorçage (125) respective et de l'autre côté à un collecteur (140) lui-même relié à ladite aiguille d'injection au moyen d'une partie de tube (148), **caractérisé en ce que** ledit dispositif comporte une pompe péristaltique comprenant un anneau (150) monté rotatif sur un vilebrequin (131) formé par un cylindre axial central (1310), ledit anneau (150) tournant autour d'un axe de rotation (Y) désaxé par rapport à l'axe de rotation (X) dudit vilebrequin (131), de sorte que l'anneau (150), au fur et à mesure qu'il tourne autour de son axe de rotation désaxé (Y), va tourner autour dudit cylindre axial central (1310) pour comprimer progressivement ladite partie de tube (148) s'étendant autour dudit vilebrequin (131), en déplaçant cette compression le long de ladite partie de tube (148).

2. Dispositif selon la revendication 1, comportant au moins deux réservoirs (210) et des moyens de sélection de réservoir pour sélectionner un ou plusieurs réservoirs (210) dont le contenu sera distribué lors du prochain actionnement, lesdits moyens de sélection comportant un organe rotatif (132, 135) pourvu de moyens de came (1320, 1350) adaptés à coopérer avec les tubes (145) de chaque réservoir (210) pour ouvrir ou fermer l'écoulement de fluide à travers chaque tube (145).

3. Dispositif selon la revendication 2, dans lequel ledit organe rotatif est une roue de sélection (132) pourvue d'une came (1320).

4. Dispositif selon la revendication 3, dans lequel ladite came (1320) est formée par une projection en arc de cercle, ladite came (1320) étant interrompue par au moins un évidement (1321), de sorte que lorsque ladite came (1320) est au contact d'un tube (145), elle le pince pour couper la circulation de fluide, et lorsqu'un tube (145) se trouve face audit évidement (1321), la circulation de fluide est possible.

5. Dispositif selon la revendication 3, dans lequel ladite came (1320) est formée de manière radiale dans un trou central (1325) de ladite roue de sélection (132), lesdits tubes (145) passant à travers ledit trou central (1325), ladite came (1320) comportant une partie de plus grand diamètre (1321), de sorte que lorsqu'un tube (145) coopère avec ladite came (1320), il est pincé, et lorsqu'un tube (145) coopère avec la partie de plus grand diamètre (1321), il n'est pas pincé.

6. Dispositif selon la revendication 2, dans lequel ledit organe rotatif est un arbre de sélection (135) pourvue d'éléments de came (1350).

7. Dispositif selon la revendication 6, dans lequel lesdits éléments de came (1350) coopèrent avec un organe de pincement (1360) pourvu de plusieurs lames flexibles, une pour chaque tube (145) de réservoir, de sorte que lorsqu'un élément de came (1350) déforme une lame flexible de l'organe de pincement, celle-ci pince son tube respectif (145) pour couper la circulation de fluide.

8. Dispositif selon l'une quelconque des revendications précédentes, comportant un actionneur rotatif unique (130) qui, lorsqu'il tourne dans un premier sens de rotation, active la sélection du ou des réservoir(s) (210) à distribuer, et qui, lorsqu'il tourne dans le sens inverse, actionne ladite pompe péristaltique (150).

9. Dispositif selon la revendication 8, dans lequel ledit actionneur (130) comporte un axe central (1300) qui s'étend dans une ouverture oblongue (1500) du corps, formant ainsi une roue flottante.

10. Dispositif selon l'une quelconque des revendications précédentes, dans lequel chaque réservoir contient entre 1 et 10 ml de produit fluide, avantageusement environ 3 ml.

11. Dispositif selon l'une quelconque des revendications précédentes, dans lequel ledit dispositif comporte un module électronique (300).

12. Dispositif selon la revendication 11, dans lequel ledit module électronique (300) comprend une alimentation, notamment une pile ou un accumulateur, un micro-processeur, des moyens de stockage, des moyens de réception et/ou d'émission de signaux et un moteur.

13. Dispositif selon la revendication 11 ou 12, dans lequel ledit module électronique (300) est réutilisable et assemblé de manière amovible sur le dispositif.

## Patentansprüche

1. Vorrichtung zum Injizieren eines Fluidprodukts, umfassend einen Körper, der dazu bestimmt ist, mit einer Injektionsstelle (SI) in Kontakt zu gelangen, zumindest ein Reservoir (210) für das Fluidprodukt, eine Injektionsnadel (120), die dazu bestimmt ist, in die Injektionsstelle (SI) einzudringen, um den Inhalt eines oder mehrerer Reservoirs (210) zu injizieren, eine dem jeweiligen Reservoir (210) zugeordnete Anstechnadel (125), die dazu bestimmt ist, vor Abgabe des Fluidprodukts in das genannte Reservoir (210) einzudringen, wobei jedes Reservoir (210) einen Schlauch (145) aufweist, der auf der einen Seite mit seiner jeweiligen Anstechnadel (125) und auf der anderen Seite mit einer Sammeleinrichtung (140) verbunden ist, die ihrerseits über einen Schlauchabschnitt (148) mit der Injektionsnadel verbunden ist,
**dadurch gekennzeichnet, dass** die Vorrichtung eine Peristaltikpumpe mit einem Ring (150) umfasst, der drehbar auf einer Kurbelwelle (131) gelagert ist, die aus einem zentralen axialen Zylinder (1310) gebildet ist, wobei sich der Ring (150) um eine Drehachse (Y) dreht, die gegenüber der Drehachse (X) der Kurbelwelle (131) versetzt angeordnet ist, so dass sich der Ring (150) mit fortschreitender Drehung um seine versetzt angeordnete Drehachse (Y) um den zentralen axialen Zylinder (1310) dreht, um den sich um die Kurbelwelle (131) erstreckenden Schlauchabschnitt (148) allmählich zu komprimieren und dabei diese Komprimierung entlang des Schlauchabschnitts (148) fortzubewegen.

2. Vorrichtung nach Anspruch 1, umfassend zumindest zwei Reservoirs (210) und eine Reservoirauswahleinrichtung zum Auswählen eines oder mehrerer Reservoirs (210), deren Inhalt bei der nächsten Betätigung ausgegeben wird, wobei die Auswahleinrichtung ein Drehglied (132, 135) mit Steuerkurvenelementen (1320, 1350) umfasst, die dazu ausgelegt sind, mit den Schläuchen (145) jedes Reservoirs (210) zusammenzuwirken, um die Fluidströmung durch jeden Schlauch (145) freizugeben bzw. zu sperren.

3. Vorrichtung nach Anspruch 2, wobei das Drehglied ein Auswahlrad (132) ist, das mit einer Steuerkurve (1320) versehen ist.

4. Vorrichtung nach Anspruch 3, wobei die Steuerkurve (1320) aus einem kreisbogenförmigen Vorsprung gebildet ist, wobei die Steuerkurve (1320) durch zumindest eine Aussparung (1321) unterbrochen ist, so dass dann, wenn die Steuerkurve (1320) mit einem Schlauch (145) in Kontakt steht, sie diesen zum Unterbrechen der Fluidzirkulation zusammendrückt, und dass dann, wenn ein Schlauch (145) der Aussparung (1321) gegenüberliegt, die Fluidzirkulation möglich ist.

5. Vorrichtung nach Anspruch 3, wobei die Steuerkurve (1320) radial in einer zentralen Lochung (1325) des Auswahlrades (132) ausgebildet ist, wobei die Schläuche (145) durch die zentrale Lochung (1325) hindurchgeführt sind, wobei die Steuerkurve (1320) einen Abschnitt mit größerem Durchmesser (1321) aufweist, so dass ein Schlauch (145), wenn er mit der Steuerkurve (1320) zusammenwirkt, zusammengedrückt wird, und ein Schlauch (145), wenn er mit dem Abschnitt mit größerem Durchmesser (1321) zusammenwirkt, nicht zusammengedrückt wird.

6. Vorrichtung nach Anspruch 2, wobei das Drehglied eine Auswahlwelle (135) ist, die mit Steuerkurvenelementen (1350) versehen ist.

7. Vorrichtung nach Anspruch 6, wobei die Steuerkurvenelemente (1350) mit einem Klemmelement (1360) zusammenwirken, das mit mehreren flexiblen Stegen ausgestattet ist, nämlich einem pro Reservoirschlauch (145), so dass dann, wenn ein Steuerkurvenelement (1350) einen flexiblen Steg des Klemmelements verformt, dieser seinen jeweiligen Schlauch (145) zusammendrückt, um die Fluidzirkulation zu unterbrechen.

8. Vorrichtung nach einem der vorstehenden Ansprüche, umfassend einen einzelnen Drehantrieb (130), der bei Drehung in einer ersten Drehrichtung die Auswahl des bzw. der auszugebenden Reservoirs (210) aktiviert und bei Drehung in der entgegengesetzten Richtung die Peristaltikpumpe (150) betätigt.

9. Vorrichtung nach Anspruch 8, wobei der Antrieb (130) eine Mittelachse (1300) aufweist, die in einer länglichen Öffnung (1500) des Körpers verläuft, wodurch ein lose gelagertes Rad gebildet ist.

10. Vorrichtung nach einem der vorstehenden Ansprüche, wobei jedes Reservoir zwischen 1 und 10 ml Fluidprodukt enthält, vorzugsweise etwa 3 ml.

11. Vorrichtung nach einem der vorstehenden Ansprüche, wobei die Vorrichtung ein Elektronikmodul (300) umfasst.

12. Vorrichtung nach Anspruch 11, wobei das Elektronikmodul (300) eine Stromversorgung, insbesondere eine Batterie oder einen Akku, einen Mikroprozessor, Speichermittel, Mittel zum Empfangen und/oder Senden von Signalen und einen Motor umfasst.

13. Vorrichtung nach Anspruch 11 oder 12, wobei das Elektronikmodul (300) wiederverwendbar und abnehmbar an der Vorrichtung angebracht ist.

## Claims

1. A fluid injection device comprising: a body for coming into contact with an injection site (SI); at least one fluid reservoir (210); an injection needle (120) for penetrating into said injection site (SI) so as to inject therein the contents of one or more reservoir(s) (210); and a respective priming needle (125) associated with each reservoir (210) for penetrating into said reservoir (210) before dispensing the fluid, each reservoir (210) including a tube (145) that is connected at one end to its priming needle (125), and at the other end to a manifold (140), itself connected to said injection needle by means of a portion of tube (148); said device being **characterized in that** it further comprises a peristaltic pump comprising a ring (150) that is mounted to rotate on a crankshaft (131) formed by a central axial cylinder 1310, said ring (150) turning about an axis of rotation (Y) that is offset relative to the axis of rotation (X) of said crankshaft (131), such that the ring (150), as it turns about its offset axis of rotation (Y), turns about said central axial cylinder (1310) to compress progressively said portion of tube (148) that extends around said crankshaft (131), moving this compression along said portion of tube (148).

2. A device according to claim 1, including at least two reservoirs (210) and reservoir selector means for selecting one or more reservoirs (210), the contents of which are to be dispensed during the next actuation, said selector means comprising a rotary member (132, 135) that is provided with cam means (1320, 1350) that are adapted to co-operate with the tubes (145) of the reservoirs (210) so as to open or close the flow of fluid through each tube (145).

3. A device according to claim 2, wherein said rotary member is a selector cog (132) provided with a cam (1320).

4. A device according to claim 3, wherein said cam (1320) is formed by a projection that is circularly arcuate, said cam (1320) being interrupted by at least one gap (1321), such that when said cam (1320) is in contact with a tube (145) it pinches it so as to cut off the flow of fluid, and when a tube (145) is situated facing said gap (1321) it is possible for fluid to flow.

5. A device according to claim 3, wherein said cam (1320) is formed in radial manner in a central hole (1325) of said selector cog (132), said tubes (145) passing through said central hole (1325), said cam (1320) including a larger-diameter portion (1321), such that when a tube (145) co-operates with said cam (1320) it is pinched, and when a tube (145) co-operates with the larger-diameter portion (1321) it is not pinched.

6. A device according to claim 2, wherein said rotary member is a selector shaft (135) provided with cam elements (1350).

7. A device according to claim 6, wherein said cam elements (1350) co-operate with a pinch member (1360) provided with a plurality of flexible blades, one for each reservoir tube (145), such that when a cam element (1350) deforms a flexible blade of the pinch member, said flexible blade pinches its respective tube (145) so as to cut off the flow of fluid.

8. A device according to any preceding claim, including a single rotary actuator (130) that, when it turns in a first direction of rotation, activates the selection of the reservoir(s) (210) to be dispensed, and that, when it turns in the opposite direction, actuates said peristaltic pump (150).

9. A device according to claim 8, wherein said actuator (130) includes a central pin (1300) that extends through an oblong opening (1500) of the body, thereby forming a floating cog.

10. A device according to any preceding claim, wherein each reservoir has a fluid content in the range 1 mL to 10 mL, advantageously about 3 mL.

11. A device according to any preceding claim, wherein said device includes an electronic module (300).

12. A device according to claim 11, wherein said electronic module (300) comprises: a power supply, in particular an optionally rechargeable battery; a microprocessor; storage means; signal transceiver means; and a motor.

13. A device according to claim 11 or claim 12, wherein said electronic module (300) is reusable and is assembled in removable manner on the device.
